# EUROPÄISCHE PATENTSCHRIFT

(11) **EP 1 568 332 B1**
(45) Veröffentlichungstag und Bekanntmachung des Hinweises auf die Patenterteilung: **04.05.2011**
(21) Anmeldenummer: 05103696.0
(22) Anmeldetag: 17.12.1999
(51) Int. Cl.: A61B 18/14

(54) **Elektrodenanordnung für ein chirurgisches Instrument zur elektrothermischen Koagulation im Gewebe**
Electrode assembly for a surgical instrument for carrying out an electrothermal coagulatino of tissue
Ensemble-electrode pour un instrument chirurgical de coagulation electrothermique de tissu

(30) Priorität: 18.12.1998 DE 19858599
(43) Veröffentlichungstag der Anmeldung: 31.08.2005
(62) Teilanmeldung aus: 99966974.0
(73) Patentinhaber: Celon AG Medical Instruments, 14513 Teltow (DE)
(72) Erfinder: Desinger, Kai, 12157 Berlin (DE)
(74) Vertreter: Eisenführ, Speiser & Partner

(56) Entgegenhaltungen:
- EP-A- 0 651 974
- WO-A-97/00647
- WO-A-97/17009
- DE-A1- 3 930 451
- DE-A1- 19 739 699

## Beschreibung

Die Erfindung betrifft eine Elektrodenanordnung für ein chirurgisches Instrument zur elektrothermischen Koagulation von Gewebe, welches einen Frontzylinder am distalen Ende des Instrumentes mit einer distalen Spitze enthält, mit einem proximal an Frontzylinder anschließenden Träger, und mit zwei Elektroden, die an eine Wechselspannungsquelle anschließbar sind.

Die Anwendung hochfrequenter Wechselströme (beispielsweise im Frequenzbereich von 300 KHz bis 2 MHz) zur Erzeugung hoher Temperaturen zu Gewebekoagulation und zur Gewebetrennung zu verwenden ist in der Chirurgie seit langem bekannt, in der Praxis werden zur Einbringung des HF-Stromes in das Gewebe sogenannte mono-polare Elektrodenanordnungen oder bipolare Elektrodenanordnungen eingesetzt.

Bei den monopolaren Anordnungen wird eine Elektrode - auch als Neutralelektrode bezeichnet - als großflächige Elektrode in der Nähe des Behandlungsortes auf die Haut des Patienten angesetzt und dort fixiert und geerdet, bzw. mit Masse verbunden. Eine zweite vom Operateur gehandhabte Elektrode - auch ais Aktivelektrode bezeichnet - ist mit der Wechselspannungsquelle verbunden. Die Elektrode ist in ihrer Form an die jeweilige Anwendung, insbesondere an die Größe des zu behandelnden Gewebebereiches so angepasst, dass sowohl die Operationsdauer als auch die thermische Belastung des betroffenen Organes bzw. Körperbereiches vertretbar sind und nur den gewünschten Gewebebereich koagulieren.

Bei Anordnungen zur bipolaren HF-Thermotherapie sind beide Elektroden mit einem HF-Generator verbunden und in miteinander festgelegte Abmessungen, beispielsweise auf einem isolierenden Träger angeordnet und werden vom Operateur in unmittelbarer Nähe der Behandlungsstelle platziert und in der Regel auch aktiv geführt.

Aus der WO 97/17009 ist eine bipolare Elektrodenanordnung mit einem Flüssigkeitskanal bekannt, über den Spülflüssigkeit in den Eingriffsbereich eingebracht werden kann. Zwei oder drei Elektroden sind als Konusabschnitt auf einer konusförmigen distalen Spitze des Instrumentes angeordnet, die in das Gewebe eingeführt werden kann, wobei das elektromagnetische HF-Feld sich zwischen den Elektroden ausbildet und das umgebene Gewebe koagulieren soll.

Aus der WO 96/34569 sowie den im zugehörigen internationalen Recherchenbericht genannten Dokumenten sind Systeme und Verfahren zur Koagulation von Körpergewebe unter Einhaltung einer vorberechneten maximalen Gewebstemperatur bekannt, bei denen während der eigentlichen Gewebskoagulation eine Fluidkühlung oder thermoelektrische Kühlung vorgesehen ist. Diese bekannten Anordnungen sind zur Einführung in Körperhöhlen über natürliche Zugänge gedacht.

Aus der DE 39 30 451 A1 ist eine Vorrichtung für Hochfrequenzkoagulation mit mindestens einer vorderen und einer hinteren Elektrode bekannt, die jeweils zumindest teilweise zylinderförmig und in Richtung einer gemeinsamen Längsachse hintereinander angeordnet sind sowie freiliegende Außenflächen aufweisen, die unterschiedliche Abmessungen in Richtung der Längsachse haben.

Aus der WO 97/00647 ist ein chirurgisches Instrument bekannt, das mittels einer bipolaren Elektrodenanordnung, die mit einem HF-Generator verbunden ist, Gewebe in einem elektrisch leitenden flüssigen Medium behandelt, wobei die Aktivelektrode der Elektrodenanordnung einen freiliegenden Anteil zur Gewebebehandlung und die Rückführungselektrode der Elektrodenanordnung eine freiliegende Kontaktoberfläche mit der Flüssigkeit aufweisen.

Aus der US 4,832,048 sowie aus der WO 95/10320 der WO 99/11186 oder der EP 96 945 879.3 und der W098/19613, der WO96/183 und der W081/03272 sind weiter chirurgische Instrumente bekannt, die mittels einer bipolaren Elektrodenanordnung Gewebe mittels HF-Thermotherapie behandeln.

Die bekannten chirurgischen Instrumente zur bipolaren HF-Thermotherapie sind oftmals aufwendig in der Herstellung, und sie besitzen für die verschiedenen Anwendungsgebiete oftmals Nachteile, die oftmals zu einer lokal ungenauen Gewebebehandlung führen, die insbesondere das zu behandelnde Gewebe teilweise nicht erreicht, bzw. gutartiges Gewebe thermisch überlastet.

Aufgabe der Erfindung ist es daher, eine Elektrodenanordnung für ein chirurgisches Instrument der eingangs genannten Art derart weiterzubilden, dass es einfach herstellbar und einsetzbar ist und eine präzise lokalisierbare Behandlung des Gewebes - bei gleichzeitiger Schonung des umgebenden gesunden Gewebes - ermöglicht.

Diese Aufgabe wird gelöst durch eine Elektrodenanordnung für ein chirurgisches Instrument zur elektrothermischen Koagulation von Gewebe gemäß Anspruch 1.

Die Vorteile der Erfindung liegen insbesondere darin, dass die Elektrodenanordnung Gerät besonders einfach aufgebaut ist, wobei nämlich der Frontzylinder eine Elektrode bildet, und dass der über ein Isolatorelement isolierte, anschließende Außenleiter die zweite Elektrode bildet, so dass die Wechselspannungsquelle über den stabförmigen Innenleiter, der durch einen Isolationsschlauchaußenleiter getrennt ist, an den Frontzylinder und von außen direkt an den Außenleiter anschließbar ist.

Je nach Einsatzzweck läßt sich die Elektrodenanordnung für ein Instrument flexibel ausbilden, so dass dann Innenleiter, Außenleiter, der Isolationsschlauch und ggf. auch das Isolationselement aus einem elastischen Material bestehen. Bei einem derartigen flexiblen chirurgischen Instrument lässt sich die bipolare Elektrodenanordnung unter Umständen leichter an den speziellen Behandlungsort verbringen. Alternativ lassen sich jedoch auch Innenleiter und Außenleiter starr und geradlinig ausbilden, wobei dann Frontzylinder und Außenleiter koaxial zueinander fluchtend angeordnet sind und dann durch eine geradlinige Translationsbewegung an den Behandlungsort verbracht werden können. Bei bestimmten Behandlungsorten kann es auch besonders vorteilhaft sein, das Instrument in Längsrichtung abzuwinkeln.

In allen Ausführungsformen besitzt der Außenleiter und der Frontzylinder im wesentlichen denselben Außendurchmesser, um eine behinderungsfreie Gleitbewegung der Elektrodenanordnung im Gewebe zu realisieren.

Bevorzugt bildet der Frontzylinder über seinen axialen Längenabschnitt, welcher nicht vom Isolationselement bedeckt ist, die erste Elektrode und der Außenleiter bildet über den vollen axialen Längenabschnitt, soweit dieser nicht vom Isolationselement bedeckt ist, die zweite zylindrische Elektrode. Die axiale Länge der Elektroden ist bevorzugt größer als die axiale Länge des Isolationselementes und auch größer als der Außendurchmesser des Frontzylinders und des Außenleiters. Bevorzugt beträgt die Länge des Außenleiters ein Mehrfaches der Länge des Frontzylinders. Wenn bei dieser Ausführungsform das an die Außenfläche des Instrumentes angrenzende Gewebe koaguliert ist und dadurch hochohmig wird, so kann sich bei dieser Ausführungsform der Erfindung das elektromagnetische Feld nach außen in angrenzende Gewebebereich hin verlagern, weil eine entsprechend lange zweite Elektrode vorhanden ist, so dass das elektromagnetische Feld, wenn an die Außenfläche angrenzend das Gewebe hochohmig geworden ist, radial nach außen wandern kann und dabei immer noch auf der zweiten Elektrode endet. Bei dieser Ausführungsform ist es also möglich, eine definiert in das Gewebe hineinwandernde Koagulation zu realisieren, die zum Ende kommt, wenn sich das Feld von der ersten Elektrode bis zum proxialen Ende der zweiten Elektrode erstreckt.

Umgekehrt hat sich gezeigt, dass der Beginn der Koagulation dann optimal ist, wenn die beiden Elektroden voneinander einen relativ geringen axialen Abstand voneinander besitzen, der in etwa die Größenordnung des Außendurchmessers aufweist oder nur geringfügig größer ist.

Gemäß der Erfindung sind Frontzylinder, der mit dem Innenleiter in der elektrischen Verbindung steht, und der Außenleiter durch einen isolierenden Ringkörper voneinander getrennt. Gemäß der Erfindung ist der isolierende Ringkörper aus lichtdurchlässigem oder teilweise lichtdurchlässigem Material hergestellt, und in dem Ringkörper wird eine Lichtquelle angeordnet, welche ihr Licht durch den Ringkörper hindurch - bevorzugt als Streulicht - nach außen abgibt. Gemäß der Erfindung besitzt der Innenleiter einen Hohlkanal, der in den isolierenden Ringkörper endet und einen Lichtwellenleiter aufnimmt. Der Innenleiter ist im Bereich des Ringkörpers radial bis in den Faserkern des Lichtwellenleiters hinein mit Einschnitten versehen, so dass Licht radial an diesen Einschnitten aus dem Lichtwellenleiter austritt und durch Ringkörper hindurch diejenige Zone der bipolaren Elektrodenanordnung für den behandelnden Arzt sichtbar macht, welche zwischen den beiden Elektroden liegt, in der also die Koagulation des Gewebes jeweils erfolgt. Bei dünnwandigem Körper sieht der behandelnde Arzt also unmittelbar mit seinem Auge stets diejenige Stelle, an der koaguliert wird. Es ist dadurch eine besonders genaue lokale Behandlung des Gewebes möglich. Die Spitze des Frontzylinders läßt sich je nach Bedarf kegelförmig oder keilförmig ausbilden.

Bei einer bevorzugten Ausführungsform der Erfindung wird der Frontzylinder als einstückiges Metallrohr oder Metallstab verwirklicht, dessen distales Ende angespitzt ist. Ein distaler Abschnitt des Metallrohres oder Metallstabes bildet die erste Elektrode. Daran angrenzend wird eine Isolierschicht auf den Träger aufgebracht, und im proximalen Bereich dieser Isolierschicht wird dann eine zylindrische Metallschicht auf die Isolierschicht abgelegt und bildet die zweite, zylindrische Elektrode. Die isolierschicht lässt sich durch einen Kunststoffschlauch verwirklichen, auf den - als zweite Elektrode - eine Metallbeschichtung aufgebracht ist. Der metallische Träger mit distaler Spitze stellt eine bipolare Elektrodenanordnung in Form einer Kanüle oder Nadel dar und eignet sich besonders zur Therapie von erweiterten Endgefäßen wie z. B. Besenreiservarizen. Die Elektrodenanordnung wird mit ihrer Spitze in Längsrichtung in das erweiterte Gefäß eingestochen. Bei Aktivierung der HF-Leistung koaguliert das Blut und die Gefäßwand primär um die erste Elektrode. Dabei zieht sich das Gefäß zusammen, so dass ein Verschluss erzielt wird, mit der Folge, dass dann kein Blut mehr in das Gefäß fließen kann, wodurch dieses nicht mehr durch die Haut zu erkennen ist und der gewünschte kosmetische Effekt erzielt wird.

Besonders vorteilhaft werden Isolatorschichten, die bei den bipolaren Elektrodenanordnungen Verwendung finden, aus Keramikmaterial eingesetzt. Der Vorteil dieses Materials ist, dass es eine hohe mechanische Festigkeit bietet und mittels einer elektrolytischen Anodisierung (Eloxierung) beispielsweise auf Titan in Form von Titanoxid, oder bei Aluminium in Form von Aluminiumoxid in einfacher Weise erzeugen lässt. Die Schichtdicke hängt ab von der bei der Elektrolyse eingesetzten elektrischen Spannung. Statt Titan eignen sich auch verschiedene Titanlegierungen als Ausgangsmaterial, auf den durch anodisches Oxidieren die Keramikschicht erzeugt wird. Um eine derartige vollständige oder partielle Beschichtung von Titan oder geeigneten Titanlegierungen oder Aluminium vorzunehmen, wird zuerst der entsprechende Metallkörper chemisch vorgereinigt, um fettfreie und oxidfreie Oberflächen zu erhalten. Im Anschluss daran werden die nicht zu beschichtenden Stellen maskiert. Die Maskierung kann durch spezielle Lacke oder Schichten aber auch durch Schrumpfschläuche verwirklicht werden. Zur anodischen Aufbringung einer keramischen Schicht ist das Ausgangsmaterial, also Titan, Titanlegierungen oder Aluminium, elektrisch zu kontaktieren und als Anode mit Spannung zu beaufschlagen.

Um beispielsweise - auf der Basis von Titan als Ausgangmaterial - eine Titanoxid-Keramikschicht aufzubringen, sind folgende Maßnahmen zu treffen: Um das Titan an seiner Oberfläche in seine Ionenphase zu überführen, ist eine entsprechend molare Säure in wässriger Lösung einzusetzen. Die in Frage kommenden molaren Lösungen liegen zwischen 0,1 bis 1 Molar H₂SO₄ (Schwefelsäure) bzw. H₃PO₄ (Phosphorsäure). Durch anlegen einer entsprechenden Gleichspannung scheidet sich an der Anode, der hier zu beschichtenden Titanelektrode, Sauerstoff ab und verbindet sich mit der ionisierten Titanoberfläche und bildet sich zu Titanoxid um. Die einzusetzenden Gleichspannungen und Ströme liegen je nach Schichtdicke zwischen 10V und 500V bei maximalen Strömen von 1 A. Dadurch durchläuft der Oxidationsprozess mehrere Oxidationsstufen (Titanoxide) je nach Länge des Prozesses. Die mit diesen Verfahren zu erzielenden Schichtdicken liegen in der Größenordnung von 20 bis 30 µm. Mit Hilfe der über die interferenzfarben darzustellenden Schichtdicken wird durch die unterschiedliche Lichtbrechung an der Grenzfläche zum Metall (die Oxidschicht ist transparent) diese proportional über ein Farbspektrum darstellbar. Mit dieser Methode lassen sich spezielle paramagnetische Elektroden aus Titan - oder Titanlegierungen wie TiAl₆V₄ - effizient mit einer dielektrischen Keramikschicht variabel hinsichtlich Schichtdicke und/oder Farbe versehen.

Neben den guten dielektrischen Eigenschaften der auf diese Weise erzeugten Keramikschichten sind auch die tribologischen Eigenschaften hevorragend geeignet, um ggf. Abriebfestigkeiten und Oberflächengüte zu erhöhen. Diese farbigen Keramikschichten eignen sich auch zur stabilen Markierung von Nadeln, Kanülen oder Sonden. Durch die Wahl der Schichtdicke ist eine proportionale Interferenzfarbe zu wählen. Damit können Farben von Grau, Gold, Violett bis hin zu Blau eingestellt werden.

Gemäß einer weiteren bevorzugten Ausführungsform der Erfindung besitzt mindestens eine der Anschlussleitungen, die zum Anschließen der Elektroden dienen, an ihrem Ende einen Abschnitt aus Federmetall, bevorzugt Federdraht, der eine solche Formgebung aufweist, dass er sich im Hohlkanal - innerhalb der Elektroden - radial nach außen gegen die Innenfläche der Elektroden verklemmt und dadurch den elektrischen Kontakt ausreichend sicher und zuverlässig herstellt. Der Federmetallabschnitt der so ausgebildeten Anschlussleitungen ist bevorzugterweise zu einer Spiralfeder gewickelt, die mit einer vorgegebenen Zugspannung im Spiraldraht beaufschlagt ist, deren Wickel unter dieser Zugspannung also einen reduzierten Durchmesser aufweist, um sich von außen in einfacher Weise in den Hohlraum der Elektroden einführen zu lassen. Anschließend wird die auf den Federdraht wirkende Zugvorspannung beseitigt, die Spiralfeder erreicht dann ihren vollen Außendurchmesser und legt sich dabei von innen gegen die Innenflächen der Elektroden selbstklemmend an. Um die Einführung der entsprechenden Federabschnitte am Ende der Anschlussleitungen in einfacher Weise durchführen zu können, ist ein entsprechendes Spezialwerkzeug einsetzbar, welches es gestattet, die Spiralfeder mit reduziertem Durchmesser einzusetzen, dann die Draht-Zugvorspannung zu beseitigen und damit die Spiralfeder gegen die Innenfläche der Elektrode zur Anlage zu bringen.

Vorteilhafte Weiterbildungen der Erfindung sind durch die Merkmale der Unteransprüche gekennzeichnet.

Im folgenden werden Ausführungsbeispiele der Erfindung anhand der Zeichnung näher erläutert. Es zeigen:
- Fig. 1: einen Längsschnitt durch eine erste Ausführungsform einer bipolaren Elektrodenanordnung für ein chirurgisches Instrument;
- Fig. 2: einen Längsschnitt einer zweiten Ausführungsform einer bipolaren Elektrodenanordnung;
- Fig. 3: einen Längsschnitt einer dritten Ausführungsform einer bipolaren Elektrodenanordnung;
- Fig. 4: einen Längsschnitt einer vierten Ausführungsform einer bipolaren Elektrodenanordnung;
- Fig. 5: einen Längsschnitt durch eine fünfte Ausführungsform der bipolaren Elektrodenanordnung;
- Fig. 6: einen Längsschnitt durch eine sechste Ausführungsform der bipolare Elektrodenanordnung;
- Fig. 7: eine perspektivische Ansicht eines Frontzylinders mit einer Teilbe- schichtung mit Keramikmaterial;
- Fig. 8: einen Endabschnitt einer Anschlussleitung zum Anschließen einer Elektrode; und
- Fig. 9: eine perspektivische Ansicht einer aus einem Metallrohr gebildeten Elektrode mit einer Keramikbeschichtung und einer im Inneren des Rohres angebrachten Anschlussleitung.

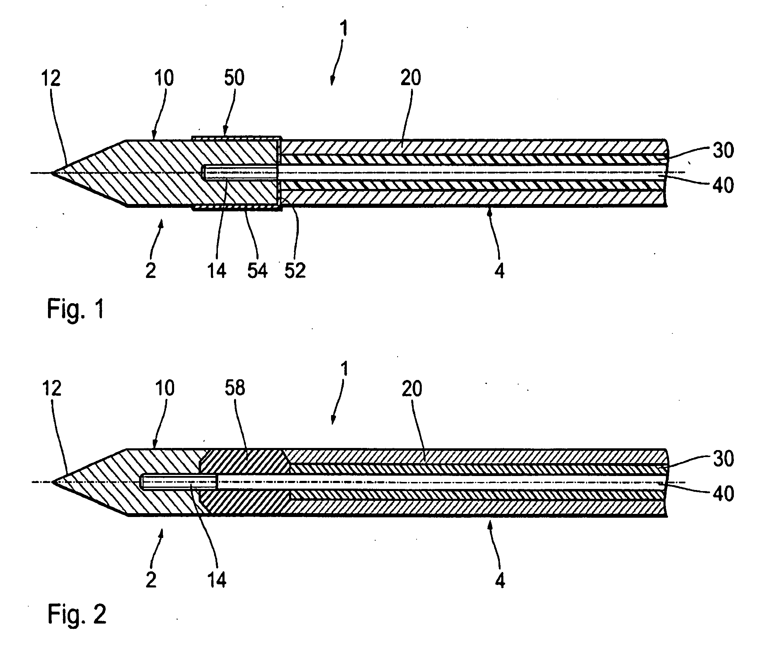

Bei den nachfolgend beschriebenen Fig. 1 bis 5 handelt es sich um Beispiele, die das Verständnis der Erfindung erleichtern sollen.

Fig. 1 zeigt einen Längsschnitt durch eine erste Ausführungsform einer bipolaren Elektrodenanordnung, die Bestandteil eines chirurgischen Instrumentes zur elektrothermischen Koagulation von Gewebe ist. Die Elektrodenanordnung enthält einen elektrisch leitenden Frontzylinder 10, der das distale, d. h. vom Benutzer des Instruments abgewandte Ende des Instruments bildet. Der Frontzylinder endet an seinem freien Ende in einer Spitze 12, die in der dargestellten Ausführungsform kegelförmig spitz ausläuft. An den Frontzylinder 10 schließt ein rohrförmiger Außenleiter 20 an, der in seinem Innenraum einen Isolationsschlauch 30 aufnimmt, durch den ein stabförmiger Innenleiter 40 hindurchverläuft. Der stabförmige Innenleiter 40 besitzt an seinem distalen Ende ein Außengewinde, weiches in ein entsprechendes inaxialer Längsrichtung verlaufendes Innengewinde verschraubbar ist und mittels dieser Schraubverbindung 14 elektrisch und mechanisch mit dem Frontzylinder 10 verbunden ist.

Zwischen dem Frontzylinder 10 und dem Außenleiter 20 ist ein Isolatorelement 50 angeordnet, weiches eine radiale Trennwand 52 zwischen Frontzylinder 10 und der distalen Stirnwand des Außenleiters 20 und des Isolationsschlauches 30 besitzt. Außen an der Trennwand 52 geht das Isolatorelement 50 in eine Hüllwand 54 über, die - in der dargestellten Ausführungsform - die Außenfläche des Frontzylinders 10 enganliegend umgibt, die jedoch in einer alternativen Ausführungsform auch - nach proximal gerichtet - die Außenfläche des Außenleiters 20 umgeben kann. Die freiliegende Außenfläche des Frontzylinders 10 bildet eine erste Elektrode 2. Die freiliegende Außenfläche des Außenleiters 20 bildet eine zweite Elektrode 4. An die beiden Elektroden wird - am proximalen Ende der Elektrodenanordnung - eine Hochfrequenz-Wechselspannungsquelle angeschlossen, wenn die bipolare Elektrodenanordnung in das zu behandelnde menschliche oder tierische Gewebe eingeführt ist und das Gewebe durch Wärmewirkung des elektrischen Feldes koaguliert werden soll.

Fig. 2 zeigt eine zweite Ausführungsform der erfindungsgemäßen bipolaren Elektrodenanordnung, bei der wiederum ein Frontzylinder 10 in eine kegelförmige distale Spitze 12 ausläuft, wobei wiederum ein stabförmiger metallischer Innenleiter von einem Isolationsschlauch 30 umgeben ist, der seinerseits von einem metallischen rohrförmigen Außenleiter 20 umgeben wird. Zwischen dem Frontzylinder 10 und der Stirnwand des Außenleiters und des Isolationsschlauches 30 ist ein Isolatorelement 50 vorgesehen, welches die Form eines Ringkörpers 58 besitzt und eine vorgegebene axiale Länge aufweist, die den Frontzylinder 10 und den Außenleiter 20 auf Abstand hält. Der Frontzylinder 10 besteht aus Metall und dient als zylindrische erste Elektrode 2. Der Außenleiter 20 ist ebenfalls aus Metall hergestellt und dient als zylindrische zweite Elektrode 4. Der Frontzylinder 10 ist mittels einer Schraubverbindung 14 mit dem Innenleiter 40 verbunden. Am proximalen Ende der Elektrodenanordnung wird zwischen dem Außenleiter 20 und dem Innenleiter 40 eine HF-Wechselspannungsquelle angeschlossen, wenn eine elektrothermische Koagulation von umgebendem Gewebe durchgeführt werden soll,

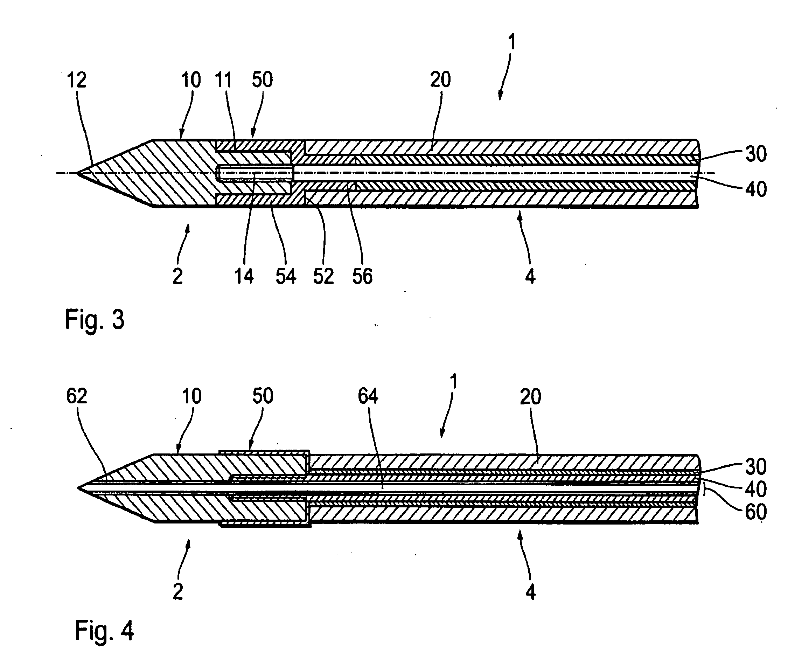

Fig. 3 zeigt eine den Fig. 1 und 2 entsprechende Ausführungsform einer bipolaren Elektrodenanordnung, bei der das Isolatorelement 50 becherförmig ausgebildet ist, wobei die zylindrische Hüllwand 54 in einer entsprechenden Ringausnehmung 11 des Frontzylinders 10 sitzt. Außerdem wird die radiale Trennwand 52 des Isolatorelements 50 nach proximal schlauchförmig - mit dem Außendurchmesser des Isolationsschlauches 30 - gegen den Isolationsschlauch 30 geführt, der eine entsprechende axiale Länge vor der distalen Stirnwand des Außenleiters 20 endet.

Fig. 4 zeigt eine vierte Ausführungsform einer erfindungsgemäßen bipolaren Elektrodenanordnung 1, die weitgehend der Anordnung gemäß Fig. 1 entspricht, wobei gleiche Teile mit denselben Bezugszeichen versehen sind. Zusätzlich zu der Anordnung gemäß Fig. 1 verläuft zentral durch den stabförmigen Innenleiter 14 und hierzu fluchtend auch durch den Frontzylinder 10 ein Hohlkanal, durch den ein Lichtwellenleiter hindurchläuft, der sichtbares Licht an die distale Spitze 12 der Elektrodenanordnung führt, wenn der Lichtwellenleiter proximal beispielsweise mit sichtbarem Laserlicht gespeist wird. Der Lichtwellenleiter 60 enthält einen Mantel 62, der den lichtleitenden Kern 64 umgibt. Um den Mantel herum kann auch noch eine Umhüllung (Cladding) vorgesehen werden.

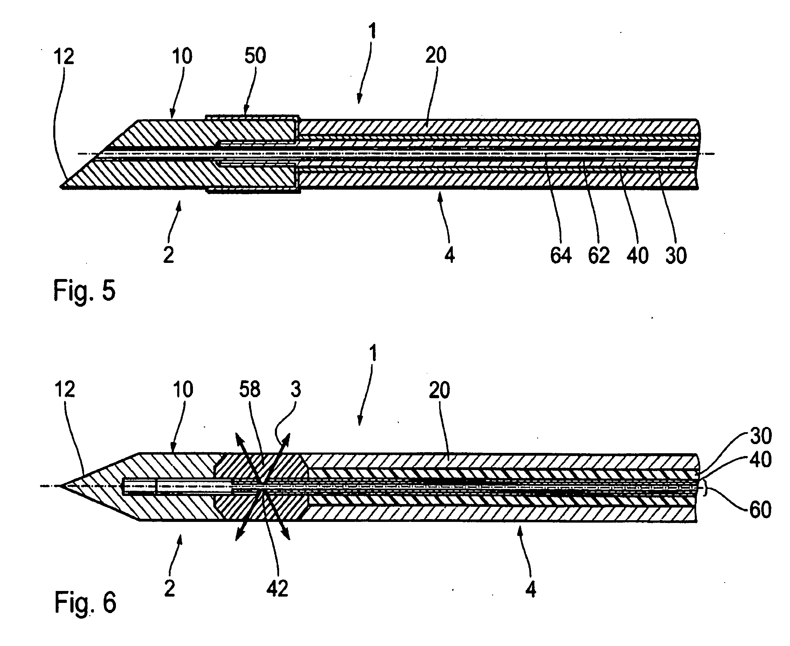

Fig. 5 zeigt eine weitere Ausführungsform der Erfindung, die weitgehend der Ausführungsform gemäß Fig. 4 entspricht wobei jedoch der Frontzylinder 10 an seinem distalen Ende eine keilförmige Spitze 12 besitzt. Wiederum ist durch den Innenleiter 40 und hieran anschließend auch durch den Frontzylinder 10 ein zentraler Hohlkanal vorhanden, durch den ein Lichtwellenleiter 60 mit seinem Mantel 62 und dem Kern 64 bis zur distalen Spitze 12 hindurchläuft und dem Benutzer der Elektrodenanordnung - insbesondere bei einer Behandlung von Gewebe in dünnwandigen Körperteilen - jeweils optisch die Position der distalen Spitze 12 im Gewebe anzeigt.

Fig. 6 zeigt eine weitere Ausführungsform der erfindungsgemäßen bipolaren Elektrodenanordnung 1, die im wesentlichen der Ausführungsform gemäß Fig. 2 oder 4 entspricht. Zwischen dem Frontzylinder 10 und der konzentrischen Anordnung aus Außenleiter 20, Isolationsstoff 30 und Innenleiter 40 ist ein Ringkörper 58 vorgesehen, durch den der Innenleiter 40 bis zum Frontzylinder 10 axial hindurchläuft. Der Innenleiter 40 weist einen zentralen Hohlkanal auf, der sich bis zum distalen Ende des Ringkörpers 58 hin erstreckt und einen Lichtwellenleiter 60 enthält. Der Ringkörper 58 ist aus transparentem oder halbtransparentem Material ausgebildet und läßt Licht nach außen hindurchtreten. Im Bereich des Ringkörpers 58 sind in den Innenleiter bis hinein in den Kern 64 des Lichtwellenleiters hinein radiale Einschliffe 42 eingebracht, mit der Folge, dass durch diese Einschliffe Licht radial aus dem Innenleiter 40 und durch den Ringkörper 58 hindurch nach außen tritt, so dass der Operateur die Position des elektrischen Feldes, welches sich zwischen der ersten Elektrode 2 und der zweiten Elektrode 4 ausbildet, wenn an den Innenleiter 40 und den Außenleiter 20 HF-Leistung eingespeist wird, optisch sichtbar gemacht werden kann. Bevorzugt ist der Ringkörper 48 aus einem solchen Material bzw. seine Oberfläche weist eine solche Struktur auf, dass das aus dem Lichtwellenleiter austretende Licht 3 als Streulicht nach außen tritt.

Alle dargestellten Ausführungsformen der bipolaren Elektrodenanordnung 1 besitzen im wesentlichen einen Kreisquerschnitt mit Radius R und über ihre Länge hinweg einen möglichst homogenen Querschnitt. Unstetigkeiten im Außendurchmesser sind möglichst gering gehalten, um die Elektrodenanordnung leichtgleitend in das Gewebe einführen zu können.

Die axiale Länge der Elektroden ist in allen dargestellten Ausführungsformen größer als der Abstand der Elektroden, der im wesentlichen in der Größenordnung des Außendurchmessers liegt. Bei dieser Dimensionierung ist eine vorteilhafte lokale Konzentration des Koagulierungsvorganges und eine ausreichende Stärke des elektrischen Feldes gegeben.

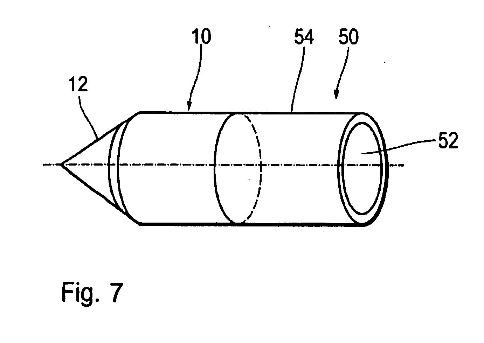

Fig. 7 zeigt einen Frontzylinder 10, der beispielsweise aus Aluminium oder Titan oder einer Titanlegierung besteht und durch anodisches Oxidieren, also einen proximalen Endbereich mit einer Keramikschicht versehen wurde, die das Isolierelement 50 bildet, dessen Trennwand 52 an dem proximalen Ende des Frontzylinders ausgebildet ist, und dessen Hüllwand 54 sich außen um einen axialen Längenabschnitt des Frontzylinders hin erstreckt.

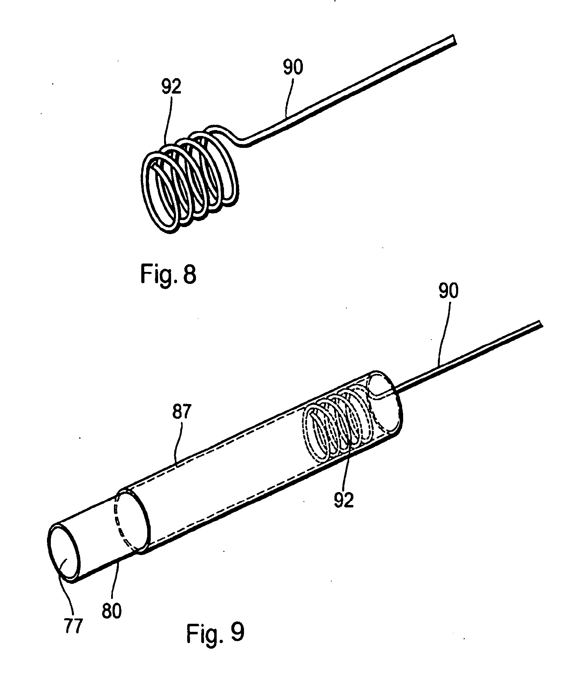

Die Fig. 8 und 9 zeigen einen als Spiralfeder 92 ausgebildeten Endabschnitt einer Anschlussleitung 90, die in einen Hohlkanal 77 eines Metallrohres 80 eingesetzt ist und unter radialem Andruck gegen die Innenfläche des Rohres 80 anliegt und dieses kontaktiert. Auf dem Metallrohr 80 ist eine Isolierschicht 87 dargestellt.

## Patentansprüche

1. Elektrodenanordnung für ein chirurgisches Instrument zur elektrothermischen Koagulation im Gewebe, enthaltend
einen elektrisch leitenden Frontzylinder (10) am distalen Ende des Instruments, mit einer distalen Spitze (12), und mit einer zylindrischen ersten Elektrode (2),
einen proximal an den Frontzylinder anschließenden rohrförmigen Außenleiter (20) mit einer zylindrischen zweiten Elektrode (4),
ein Isolatorelement (50) zwischen dem Frontzylinder (10) und dem Außenleiter (20), wobei die Elektroden (2, 4) an eine Wechselspannungsquelle anschließbar sind,
einen stabförmigen Innenleiter (40) in dem Außenleiter (20) und
einen Isolationsschlauch (30) zwischen dem Innenleiter (40) und dem Außenleiter (20), **dadurch gekennzeichnet, dass** der Innenleiter in Längsrichtung einen Hohlkanal aufweist, der in dem isolierenden Ringkörper (58) endet und einen Lichtwellenleiter (60) aufnimmt, dass der Innenleiter (40) im Bereich des Ringkörpers (58) radial bis in den Faserkem des Lichtwellenleiters hineinragende Einschnitte (42) besitzt, und die Lichtquelle bildet die durch den isolierenden Ringkörper (58) aus lichtdurchlässigem oder teilweise lichtdurchlässigem Material das seitlich aus dem Lichtwellenleiter (60) austretende Licht als Streulicht-nach außen abgibt.

2. Elektrodenanordnung für ein chirurgisches Instrument nach Anspruch 1, **dadurch gekennzeichnet, dass** die axiale Länge der Elektroden (2, 4) größer ist als der Durchmesser der Elektroden (2, 4).

3. Elektrodenanordnung für ein chirurgisches Instrument nach einem der vorstehenden Ansprüche, **dadurch gekennzeichnet, dass** die axiale Länge der Elektroden (2, 4) größer ist als die axiale Länge des vom Isolationselement (50) belegten Längenabschnitts.

4. Elektrodenanordnung für ein chirurgisches Instrument nach einem der vorstehenden Ansprüche, **dadurch gekennzeichnet, dass** die axiale Länge der Elektroden (2, 4) größer ist als der Außendurchmesser des Frontzylinders (10) bzw. des Außenleiters (20).

5. Elektrodenanordnung für ein chirurgisches Instrument nach einem der vorstehenden Ansprüche, **dadurch gekennzeichnet, dass** der axiale Abstand der Elektroden (2, 4) voneinander etwa gleich oder kleiner ist als der Außendurchmesser des Frontzylinders (10).

6. Elektrodenanordnung für ein chirurgisches Instrument nach einem der vorstehenden Ansprüche, **dadurch gekennzeichnet, dass** metallische Zylinderkörper (10), die zur Ausbildung der Elektroden (2, 4) vorgesehen sind, aus Titan oder Aluminium bestehen, und dass die Isolierschichten (50), welche auf dem metallischen Zylinderkörper (10) vorgesehen ist, durch anodische Oxidation der Metalloberfläche im Elektrolytbad aufgebracht ist.

7. Elektrodenanordnung für ein chirurgisches Instrument nach Anspruch 6, **dadurch gekennzeichnet, dass** der metallische Zylinderkörper (10) als Anode in einem Elektrolyten, beispielsweise H₂SO₄ (Schwefelsäure) bzw. H₃PO₄ (Phosphorsäure), und eine Hilfselektrode als Kathode geschaltet und an eine entsprechende Gleichspannung gelegt sind.

8. Elektrodenanordnung für ein chirurgisches Instrument nach Anspruch 6 oder 7, **dadurch gekennzeichnet, dass** Teile des metallischen Zylinderkörpers (10), die nicht mit einer Oxidationsschicht versehen werden, mit Kunststoffschichten oder Kunststoffschläuchen maskiert werden, und dass anschließend die Oxidation des Aluminiums oder Titans mittels Elektrolyse erfolgt.

9. Elektrodenanordnung für ein chirurgisches Instrument nach einem der vorstehenden Ansprüche, **dadurch gekennzeichnet, dass** mindestens eine der Anschlussleitungen (90) zum Anschließen der Elektroden (2, 4) am einen Ende einen Abschnitt aus Federmetall aufweist, der im Hohlkanal radial auswärts gegen die Innenfläche der Elektroden (2, 4) verklemmbar ist.

10. Elektrodenanordnung für ein chirurgisches Instrument nach Anspruch 9, **dadurch gekennzeichnet, dass** der Federmetall-Abschnitt des/der Anschlussleitungen zu einer Spiralfeder (92) gewickelt ist, die im Hohlkanal (77) unter Ausübung radialer Kräfte nach außen gegen die Innenfläche der Elektroden (2, 4) selbstklemmend anlegbar ist.

## Claims

1. Electrode arrangement for a surgical instrument for electrothermal coagulation in tissue, comprising:
an electrically conducting front cylinder (10) at the distal end of the instrument, with a distal tip (12), and with a cylindrical first electrode (2),
a tubular external conductor (20) connected proximally to the front cylinder with a cylindrical second electrode (4),
an insulator element (50) between the front cylinder (10) and the external conductor (20), wherein the electrodes (2, 4) can be connected to an alternating current source, a rod-like inner conductor (40) in the external conductor (20) and an insulation hose (30) between the inner conductor (40) and the external conductor (20), **characterised in that** the inner conductor in longitudinal direction comprises a hollow channel,
which ends in the insulating annular body (58) and holds a fibre optic cable (60), **in that** the inner conductor (40) in the region of the annular body (58) has notches (42) projecting radially into the fibre core of the fibre optic cable and forms the light source which emits outwardly through the insulating annular body (58) made of light-permeable or partly light-permeable material the light emerging laterally from the fibre optic cable (60) as scattered light.

2. Electrode arrangement for a surgical instrument according to claim 1, **characterised in that** the axial length of the electrodes (2, 4) is greater than the diameter of the electrodes (2, 4).

3. Electrode arrangement for a surgical instrument according to one of the preceding claims, **characterised in that** the axial length of the electrodes (2, 4) is greater than the axial length of the longitudinal section occupied by the insulation element (50).

4. Electrode arrangement for a surgical instrument according to one of the preceding claims, **characterised in that** the axial length of the electrodes (2, 4) is greater than the external diameter of the front cylinder (10) or the external conductor (20).

5. Electrode arrangement for a surgical instrument according to one of the preceding claims, **characterised in that** the axial distance of the electrodes (2, 4) from one another is approximately the same or smaller than the external diameter of the front cylinder (10).

6. Electrode arrangement for a surgical instrument according to one of the preceding claims, **characterised in that** metal cylinder bodies (10) which are provided to form the electrodes (2, 4) are made of titanium or aluminium, and **in that** the insulation layer (50), which is provided on the metal cylinder body (10), is applied by anodic oxidation of the metal surface in an electrolyte bath.

7. Electrode arrangement for a surgical instrument according to claim 6, **characterised in that** the metal cylinder body (10) is connected as an anode in an electrolyte, for example H₂SO₄ (sulphuric acid) or H₃PO₄ (phosphoric acid) and an auxiliary electrode is connected as a cathode and a corresponding direct current is applied.

8. Electrode arrangement for a surgical instrument according to claim 6 or 7, **characterised in that** parts of the metal cylinder body (10), which are not provided with an oxidation layer, are masked with plastic layers or plastic hoses, and **in that** subsequently the oxidation of the aluminium or titanium is performed by means of electrolysis.

9. Electrode arrangement for a surgical instrument according to one of the preceding claims, **characterised in that** at least one of the connection lines (90) for connecting the electrodes (2, 4) to one end has a section made of spring metal, which can be clamped in the hollow channel radially outwards against the inner surface of the electrodes (2, 4).

10. Electrode arrangement for a surgical instrument according to claim 9, **characterised in that** the spring metal section of the connection line(s) is wound into a spiral spring (92), which in the hollow channel (77) with the exertion of radial forces outwards can be applied against the inner surface of the electrodes (2, 4) in a self-clamping manner.

## Revendications

1. Ensemble-électrode pour un instrument chirurgical de coagulation électrothermique de tissu, comprenant
- un cylindre frontal (10) électriquement conductible à l'extrémité distale de l'instrument, avec une pointe distale (12) et avec une première électrode cylindrique (2),
- un conducteur externe (20) en forme de tube, attenant de façon proximale au cylindre frontal, avec une deuxième électrode cylindrique (4),
- un élément isolateur (50) entre le cylindre frontal (10) et le conducteur externe (20), les électrodes (2, 4) pouvant être raccordées à une source de tension alternative,
- un conducteur interne (40) en forme de baguette dans le conducteur (20), et
- un tuyau (30) d'isolation entre le conducteur interne (40) et le conducteur externe (20), **caractérisé en ce que** le conducteur interne présente un canal creux dans le sens de la longueur, qui se termine dans le corps annulaire (58) isolant et abrite un guide (60) d'ondes lumineuses, **en ce que** le conducteur interne (40) possède des encoches (42) s'engageant de manière radiale jusque dans le coeur de la fibre du guide d'ondes lumineuses dans la zone du corps annulaire (58), et constitue la source lumineuse qui émet vers l'extérieur, à travers le corps annulaire (58) isolant en matière translucide ou partiellement translucide, la lumière provenant latéralement du guide (60) d'ondes lumineuses en tant que lumière diffusée.

2. Ensemble-électrode pour un instrument chirurgical selon la revendication 1, **caractérisé en ce que** la longueur axiale des électrodes (2, 4) est supérieure au diamètre des électrodes (2, 4).

3. Ensemble-électrode pour un instrument chirurgical selon l'une des revendications précédentes, **caractérisé en ce que** la longueur axiale des électrodes (2, 4) est supérieure à la longueur axiale du tronçon longitudinal occupé par l'élément (50) d'isolation.

4. Ensemble-électrode pour un instrument chirurgical selon l'une des revendications précédentes, **caractérisé en ce que** la longueur axiale des électrodes (2, 4) est supérieure au diamètre extérieur du cylindre frontal (10) ou du conducteur externe (20).

5. Ensemble-électrode pour un instrument chirurgical selon l'une des revendications précédentes, **caractérisé en ce que** l'écart axial entre les électrodes (2, 4) est inférieur ou égal au diamètre extérieur du cylindre frontal (10).

6. Ensemble-électrode pour un instrument chirurgical selon l'une des revendications précédentes, **caractérisé en ce que** le corps cylindrique (10) métallique servant à former les électrodes (2, 4) est en titane ou en aluminium, et **en ce que** la couche isolante prévue sur le corps cylindrique (10) est apposée par oxydation anodique de la surface métallique dans le bain électrolytique.

7. Ensemble-électrode pour un instrument chirurgical selon la revendication 6, **caractérisé en ce que** le corps cylindrique (10) métallique est mis à l'anode dans un électrolyte, par exemple H₂SO₄ (acide sulfurique) ou H₃PO₄ (acide phosphorique), une électrode auxiliaire étant mise à la cathode et soumise à une tension correspondante du courant continu.

8. Ensemble-électrode pour un instrument chirurgical selon la revendication 6 ou 7, **caractérisé en ce que** des parties du corps cylindrique (10) métallique, qui ne sont pas recouvertes d'une couche d'oxydation, sont masquées par des couches de plastique ou des tuyaux en plastique, et **en ce que** l'oxydation de l'aluminium ou du titane intervient ensuite par électrolyse.

9. Ensemble-électrode pour un instrument chirurgical selon l'une des revendications précédentes, **caractérisé en ce qu'**au moins une des lignes (90) pour raccorder les électrodes (2, 4) présente à une extrémité une partie en métal élastique qui peut être coincée de manière radiale vers l'extérieur contre la face interne des électrodes (2, 4) dans le canal creux.

10. Ensemble-électrode pour un instrument chirurgical selon la revendication 9, **caractérisé en ce que** la partie en métal élastique de la/des ligne(s) a la forme d'un ressort spiral (92) qui, sous l'action de forces radiales vers l'extérieur, peut être placé en auto-coincement contre la face interne des électrodes (2, 4) dans le canal creux (77).
